# EUROPEAN PATENT APPLICATION

(11) **EP 1 550 438 A2**
(43) Date of publication of application: **06.07.2005**
(21) Application number: 04078364.9
(22) Date of filing: 10.12.2004
(51) Int. Cl.: A61K 9/00, A61K 31/137, A61P 11/10

(54) **Method for preparing a medicated candy containing ambroxol and the thus obtained candy**

(30) Priority: 16.12.2003 IT MI20032462
(71) Applicant: Advance Holdings Limited, Floriana, Malta (ML)
(72) Inventor: Pifferi, Giorgio, 20147 Milano (IT)
(74) Representative: Marchi, Massimo

(57) **Abstract**

Method for preparing a candy including ambroxol, comprising (a) preparing an aqueous solution including at least one disaccharide compound having at least one carbonyl function, (b) boiling said aqueous solution under stirring to remove water until a viscous molten mass is obtained, (c) raising the temperature of the molten mass under continuous stirring until substantially all water has been removed by evaporation, (d) reducing the temperature of the molten mass under stirring, (e) adding ambroxol in powder form to the molten mass maintained under stirring, possibly along with other ingredients, (f) reducing the temperature of the molten mass under stirring, (g) extruding said mass, and (h) cutting the extruded string into single units.

Candy comprising ambroxol or a physiologically acceptable acid addition salt thereof in a sugar matrix made of a disaccharide compound having at least one carbonyl function.

## Description

This invention relates to a method for preparing a medicated candy to be sucked based on ambroxol and to the thus obtained candy.

More particularly, this invention relates to a method for preparing a candy comprising ambroxol incorporated in a sugar matrix made of at least one disaccharide compound having at least one carbonyl function.

Ambroxol [trans-4-(2-amino-3,5-dibromobenzylamino)cyclohexanol] is a well known mucolytic drug available in various compositions suitable for oral or parenteral administration. However, oral administration is by far preferred.

PCT application WO 01/05378 discloses a lozenge for the treatment of sore throat symptoms. Said lozenge has local and systemic action.

The said document reports that, in the presence of carbonyl groups, particularly formaldehyde and formaldehyde releasing compounds, ambroxol forms a product having higher molecular weight.

Moreover, an impurity due to the interaction between ambroxol and formaldehyde is specifically mentioned in the *European Pharmacopoeia* IV Ed..

This problem prompted the inventors of the above mentioned patent application to use reduced sugars, hence lacking carbonyl functions, such as sorbitol. Said inventors further report that ambroxol interacts also in the presence of sorbitol and teach to prevent said interaction by using a very specific matrix which comprises, in addition to sorbitol, also a laminar silicate (in particular talcum) and a polyethylene glycol. According to the above mentioned patent application, this is the only sugar matrix that allows preventing the interaction of ambroxol.

Subsequently, two articles have been published (Deutsche Apotheker Zeitung; 17, 2113, 2002; J. Fischer et al. Arzneim. Forsch.; 52, 256, 2002) relating to controlled studies towards placebos, randomized and statistically significant, which prove the effectiveness on sore throat symptoms of tablets containing 20 or 30 mg of ambroxol.

It is also known that one of the ways that the patients enjoy most for the therapy of sore throat symptoms is the administration of drugs formulated as common candy to be sucked, having a matrix made of at least one disaccharide compound having at least one carbonyl function such as saccharose, isomalt (*European Pharmacopoeia* IV Ed., page 1403) and maltitol (*European Pharmacopoeia* IV Ed., page 1522) and mixtures thereof.

However, in the case of ambroxol, this kind of matrix has the drawback that the preparation of a candy implies a thermal process in the presence of moisture which can favour the formation of free carbonyl groups capable of reacting with ambroxol.

The Applicant has now surprisingly found that said drawback does not occur when ambroxol is added to a molten mass of at least one disaccharide compound, having at least a carbonyl function, wherefrom water has been substantially removed.

In a first aspect, this invention therefore relates to a method for preparing a candy including ambroxol, where said method comprises the following steps:
a) preparing an aqueous solution including at least one disaccharide compound having at least one carbonyl function,
b) boiling said aqueous solution under stirring to remove water until a viscous molten mass is obtained,
c) raising the temperature of the molten mass under continuous stirring until substantially all water has been removed by evaporation,
d) reducing the temperature of the molten mass under stirring,
e) adding ambroxol in powder form to the molten mass maintained under stirring, possibly along with other ingredients,
f) reducing the temperature of the molten mass under stirring,
g) extruding said mass, and
h) cutting the extruded string into single units.

Preferably, in step a) said at least one disaccharide having at least one carbonyl function is a glucopyranosyl compound selected from the group comprising saccharose, isomalt, maltitol and mixtures thereof.

In step a), the aqueous solution can also comprise flavouring agents, dyes, sweeteners and other ingredients commonly used in food and pharmaceutical industries.

Alternatively, said flavouring agents, dyes, sweeteners and other ingredients are added in step e).

In another embodiment, part of these flavouring agents, dyes, sweeteners and other ingredients commonly used in the food and pharmaceutical industries is added in step a) and the remaining ones are added in one of the subsequent steps.

Advantageously, in step c) the molten viscous mass is heated to 140-150°C.

Preferably, in steps b) and c) water is removed by evaporation under vacuum.

Typically, in step d) temperature is reduced to 100-110°C.

Preferably, in step d) ambroxol is added as a physiologically acceptable acid addition salt thereof. Even more preferably, ambroxol is added as hydrochloride.

Advantageously, the running time of step e) shall be carefully controlled to distribute ambroxol uniformly in the molten mass without, however, heating the same longer than necessary. The person skilled in the art will readily understand that said time varies as the stirring efficiency and the temperature of the molten mass vary. As a general rule, said time shall be less than 20 minutes. Preferably, it shall range of from 5 to 15 minutes.

Preferably, in step f) the temperature of the molten mass is quickly reduced to 75-85°C.

As is well known, ambroxol is also characterized by a particularly bitter aftertaste which is particularly difficult to mask.

The Applicant has now surprisingly found that ammonium salts of glycyrrhizic acid are particularly suitable to mask the bitter aftertaste of ambroxol. The preferred salt is ammonium monoglycyrrhizinate.

For this purpose, the combined action of ammonium monoglycyrrhizinate with menthol has been found to be particularly effective.

The preferred ammonium monoglycyrrhizinate/menthol weight ratio ranges of from 0.5:10 to 5:10.

In turn, the preferred ammonium monoglycyrrhizinate/ambroxol weight ratio ranges of from 0.001:1 to 0.1:1. More preferably, the aforesaid weight ratio ranges of from 0.01:1 to 0.05:1, even more preferably, it ranges of from 0.011:1 to 0.025:1.

The candy of this invention is suitable for mucolytic therapy in place of known compositions comprising ambroxol. However, broad investigations have surprisingly shown that the candy of this invention is also effective in the treatment of sore throat symptoms.

In a second aspect, this invention also relates to a candy comprising ambroxol or a physiologically acceptable acid addition salt thereof in a sugar matrix, wherein said sugar matrix is made of a disaccharide compound having at least one carbonyl function.

The amount of the disaccharide compound in the candy of the invention is not critical.

The amount of ambroxol in the candy of this invention is preferably of from 5 to 60 mg. Even more preferably, it is of from 10 to 40 mg. Typically, the amount of ambroxol in the candy of this invention is of from 20 to 30 mg.

Typically, the physiologically acceptable acid addition salt is hydrochloride.

Preferably, the disaccharide is a glucopyranosyl compound selected from the group comprising saccharose, isomalt, maltitol, and mixture thereof.

Advantageously, said candy also comprises an ammonium salt of glycyrrhizic acid. Preferably, said salt is ammonium monoglycyrrhizinate.

In a preferred embodiment, said candy also comprises menthol.

Preferably, the ammonium monoglycyrrhizinate/menthol and ammonium monoglycyrrhizinate/ambroxol weight ratios fall within the above mentioned ranges.

The following examples further illustrate the invention, without limiting it.

### Example 1

A candy of the invention weighs 4.00 g and comprises:

| Ingredient | mg |
|---|---|
| Ambroxol hydrochloride | 20.000 |
| Isomalt | 3971.700 |
| Ammonium monoglycyrrhizinate | 0.250 |
| Menthol | 2.000 |
| Acesulphame | 6.000 |
| Indigotine (E 132) | 0.015 |

The candy was prepared as follows.

Isomalt (100 kg) and purified water (40 L) were placed into a suitable steel reactor.

The mixture was heated at the reflux temperature under stirring until it was obtained a homogeneous viscous solution. Still under stirring, the solution was gradually heated to 140-150°C and vacuum was applied in the final step to remove water. The thus obtained mass was cooled down to 100-110°C and, still under stirring, ambroxol hydrochloride (503.56 g), a hydro-alcoholic solution (126 mL) of ammonium monoglycyrrhizinate (6.29 g), an alcoholic solution (100 mL) of menthol (50.36 g), acesulphame (151.07 g) and E 132 (0.38 g) were rapidly added.

The mass was mixed at the same temperature for about 10 minutes, then temperature was quickly reduced to about 80°C.

The molten mass was transferred onto a steel plane and then forced through an extruder to form a long continuous string. The string was cut into pieces which, whilst still warm, were pressed to obtain candies having the desired weigh and shape.

The thus obtained candies did not contain any unknown ambroxol interaction products, whilst the known ones, when present, were lower than the maximum limits required by the *European Pharmacopoeia,* IV Ed.

After cooling the candies were singly wrapped in paraffin-coated paper and placed in suitable containers by means of conventional packaging machine.

### Example 2

A candy of the invention weighs 2.00 g and comprises:

| Ingredient | mg |
|---|---|
| Ambroxol hydrochloride | 20.000 |
| Maltilol | 1957.799 |
| Isomalt | 20.000 |
| Ammonium monoglycyrrhizinate | 0.235 |
| Menthol | 1.950 |
| Indigotine (E 132) | 0.016 |

The candy was prepared as follows:

Maltilol (19.578 kg) and purified water (6.5 L) were placed into a suitable steel container.

The mixture was heated under stirring to 80-90°C and indigotine (163 mg) was added.

The mixture was transferred into a steel reactor where it was heated to 130-140°C under stirring and vacuum was applied until substantially complete removal of water.

The hot viscous mass (paste) was transferred in a kneading machine and the temperature of the mass was allowed to decrease to 100-110°C. At this temperature the viscous mass was added with a homogeneous mixture of Ambroxol hydrochloride (200 g), isomalt (200 g), an alcoholic solution (32.5 g) of menthol (19.5 g) and an aqueous solution (44.4 g) of ammonium mono glycyrrhizinate (2.35), under mixing while the temperature was allowed to decrease.

Then the viscous mass was poured on a steel table where it was still mixed and the temperature was allowed to decrease.

The subsequent extrusion formed a continuous string which was cut into warm pieces.

The warm pieces were pressed to obtain candies having the desired weigh and shape.

Finally the still warm candies were cooled by a stream of forced air and packaged in blisters.

The thus obtained candies were pleasant-tasting and complied with the requirements of the *European Pharmacopoeia,* IV Ed.

### Assay 1

### Evaluation of the activity on sore throat symptoms

A double blind trial was carried out on a group of 20 patients with acute non complicated sore throat who were divided randomly into two groups of 10 patients each.

The first group was treated with placebo, the second one was treated with the candy of Example 1.

At the time of the evaluation, patients were asked to grade pain on a scale from 0 (= no pain) to 5 (= highly intense pain).

The evaluation was conducted before treatment and 30, 60, 120 and 180 minutes after treatment.
Results (pain intensity vs. placebo):
Candy: 30 minutes (52%), 60 minutes (36%), 120 minutes (31%), 180 minutes (31 %);

## Claims

1. A method for preparing a candy including ambroxol, where said method comprises the following steps:
a) preparing an aqueous solution including at least one disaccharide compound having at least one carbonyl function,
b) boiling said aqueous solution under stirring to remove water until a viscous molten mass is obtained,
c) raising the temperature of the molten mass under continuous stirring until substantially all water has been removed by evaporation,
d) reducing the temperature of the molten mass under stirring,
e) adding ambroxol in powder form to the molten mass maintained under stirring, possibly along with other ingredients,
f) reducing the temperature of the molten mass under stirring,
g) extruding said mass, and
h) cutting the extruded string into single units.

2. A method according to claim 1, wherein said at least one disaccharide having at least one carbonyl function is a glucopyranosyl compound selected from the group comprising saccharose, isomalt, maltitol and mixtures thereof.

3. A method according to claim 1 or 2, wherein in step a), said aqueous solution can also comprise flavouring agents, dyes, sweeteners and other ingredients commonly used in the food and pharmaceutical industries.

4. A method according to claim 1 or 2, wherein said flavouring agents, dyes, sweeteners and other ingredients commonly used in the sweets industry are added in step e).

5. A method according to claim 3, wherein part of these flavouring agents, dyes, sweeteners and other ingredients commonly used in the food and pharmaceutical industries is added in step a) and the remaining ones are added in one of the subsequent steps.

6. A method according to any one of the preceding claims 1 to 5, wherein in step c) said viscous molten mass is heated to 140-150°C.

7. A method according to any one of the preceding claims 1 to 6, wherein in steps b) and c) water is removed by evaporation under vacuum.

8. A method according to any one of the preceding claims 1 to 7, wherein in step d) the temperature is reduced to 100-110°C.

9. A method according to any one of the preceding claims 1 to 8, wherein in step e) ambroxol is added as a physiologically acceptable acid addition salt thereof.

10. A method according to claim 9, wherein ambroxol is added as hydrochloride.

11. A method according to any one of the preceding claims 1 to 10, wherein the running time of step e) is carefully controlled to distribute ambroxol uniformly in the molten mass.

12. A method according to any one of the preceding claims 1 to 11, wherein in step f) the temperature of said molten mass is rapidly reduced to 75-85°C.

13. A method according to any one of the preceding claims 1 to 12, wherein in step e) an ammonium salt of glycyrrhizic acid is also added.

14. A method according to claim 13, wherein said added salt is ammonium monoglycyrrhizinate.

15. A method according to any one of the preceding claims 1 to 14, wherein in step e) menthol is added as well.

16. A method according to claim 15, wherein the ammonium monoglycyrrhizinate/menthol weight ratio ranges of from 0.5:10 to 5:10.

17. A method according to any one of the preceding claims 9 to 11, wherein the ammonium monoglycyrrhizinate/ambroxol weight ratio ranges of from 0.001:1 to 0.1:1.

18. A method according to claim 17, wherein said weight ratio ranges of from 0.01:1 to 0.05:1.

19. A method according to claim 18, wherein said weight ratio ranges of from 0.011:1 to 0.025:1.

20. A candy comprising ambroxol or a physiologically acceptable acid addition salt thereof in a sugar matrix, wherein said sugar matrix is made of a disaccharide compound having at least one carbonyl function.

21. A candy according to claim 20, wherein said physiologically acceptable acid addition salt is hydrochloride.

22. A candy according to claim 20 or 21, wherein the amount of ambroxol ranges of from 5 to 60 mg.

23. A candy according to claim 22, wherein the amount of ambroxol ranges of from 10 to 30 mg.

24. A candy according to claim 23, wherein the amount of ambroxol ranges of from 15 to 25 mg.

25. A candy according to any one of the preceding claims 20 to 24, wherein said disaccharide is a glucopyranosyl compound selected from the group comprising saccharose, isomalt, maltilol, and mixture thereof.

26. A candy according to any one of the preceding claims 20 to 25, further comprising an ammonium salt of glycyrrhizic acid.

27. A candy according to claim 26, wherein said ammonium salt is ammonium monoglycyrrhizinate.

28. A candy according to any one of the preceding claims 20 to 27, further comprising menthol.

29. A candy according to any one of the preceding claims 20 to 28, wherein the ammonium monoglycyrrhizinate/menthol weight ratio ranges of from 0.5:10 to 5:10.

30. A candy according to any one of the preceding claims 20 to 29, wherein the ammonium monoglycyrrhizinate/ambroxol weight ratio ranges of from 0.001:1 to 0.1:1.

31. A candy according to claim 30, wherein said weight ratio ranges of from 0.01:1 to 0.05:1.

32. A candy according to claim 31, wherein said weight ratio ranges of from 0.011:1 to 0.025:1.
